# EUROPÄISCHE PATENTSCHRIFT

(11) **EP 0 672 651 B1**
(45) Veröffentlichungstag und Bekanntmachung des Hinweises auf die Patenterteilung: **09.12.1998**
(21) Anmeldenummer: 95102766.3
(22) Anmeldetag: 27.02.1995
(51) Int. Cl.: C07C 253/30, C07C 255/37

(54) **Verfahren zur Herstellung von 4-Fluoralkoxyzimtsäurenitrilen**
Process for the preparation of 4-fluoroalkoxycinnamonitriles
Procédé pour la préparation de 4-fluoroalcoxycinnamonitriles

(30) Priorität: 10.03.1994 DE 4408083
(43) Veröffentlichungstag der Anmeldung: 20.09.1995
(73) Patentinhaber: Clariant GmbH, 65929 Frankfurt am Main (DE)
(72) Erfinder: Beller, Matthias, Dr., D-65510 Idstein (DE); Forstinger, Klaus, Dr. Colour Chemical Ltd., Balkum Thane 400 608 (IN)

(56) Entgegenhaltungen:
- EP-A- 0 174 769
- DE-C- 4 408 083
- CHEMISCHE BERICHTE, Bd. 95, 1962 WEINHEIM DE, Seiten 967-970, G.P. SCHIEMENZ ET AL. 'Substituenteneinflüsse bei der Reaktion aromatischer Aldehyde mit Cyanessigsäure'
- JOURNAL OF ORGANIC CHEMISTRY, Bd. 48, 1983 EASTON US, Seiten 3771-3773, J.P. IDOUX ET AL. 'Aromatic Fluoroalkoxylation via Direct Aromatic Nucleophilic Substitution'

## Beschreibung

Die vorliegende Erfindung betrifft ein Verfahren zur Herstellung von 4-Fluoralkoxyzimtsäurenitrilen.

4-Fluoralkoxyzimtsäurenitrile haben technische Bedeutung als Pharmazwischenprodukt. Insbesondere sind sie als Ausgangsmaterial für Antimykotica von Bedeutung (ICI-D0870, Drugs of the Future, 18 (5), 424-427).

Bisherige Synthesen von 4-Fluoralkoxyzimtsäurenitrilen gehen beispielsweise von p-Chlorbenzonitril aus, das im ersten Reaktionsschritt mit 2,2,3,3-Tetrafluorpropanol zum 4-(2,2,3,3-Tetrafluorpropoxy)benzonitril umgesetzt wird (EP 0 472 392). Diese Reaktion wurde bereits von J. P. Idoux (J. Org. Chem. 48, 3772, 1983) beschrieben. Im zweiten Reaktionsschritt wird 4-(2,2,3,3-Tetrafluorpropoxy)benzonitril in Gegenwart von Metallhydriden wie Diisobutylaluminiumhydrid zum entsprechenden Benzaldehyd umgesetzt (JPS Sho 61-72767). Anschließend reagiert 4-(2,2,3,3-Tetrafluorpropoxy)benzaldehyd mit Ethoxycarbonyl-methanphosphonsäurediethylester zum 4-(2,2,3,3-Tetrafluorpropoxy)zimtsäureethylester, der in der vierten Stufe zur freien Zimtsäure hydrolisiert wird. Der fünfte Reaktionsschritt besteht in der Bildung des Zimtsäurechlorids das in der sechsten Reaktionsstufe mit wäßrigem Ammoniak zum 4-(2,2,3,3-Tetrafluorpropoxy)zimtsäureamid reagiert. In der siebten und letzten Stufe der Reaktionssequenz wird das genannte Zimtsäureamid in Gegenwart von Thionylchlorid oder Phosphorpentachlorid zum gewünschten Produkt umgesetzt.

Die in EP 0 472 392 beschriebene Methode zur Herstellung von 4-(2,2,3,3-Tetrafluorpropoxy)zimtsäurenitril in sieben Reaktionsstufen ist ungeeignet das Produkt in größeren Mengen technisch herzustellen. Die Nachteile dieses Verfahrens liegen insbesondere in der niedrigen Gesamtausbeute des gewünschten Produktes, der großen Anzahl von Stufen, bei denen viele Nebenprodukte, Salz- und Lösungsmittelabfälle anfallen. Problembehaftet ist auch die Verwendung von sehr teuren Reagenzien wie Metallhydriden und Ethoxycarbonylmethylphosphonsäureestern. Die Verwendung von Metallhydriden bedingt zudem besondere Sicherheitsvorkehrungen bei der technischen Durchführung des Verfahrens.

EP 174 769 beschreibt die Synthese des analogen 4-Trifluormethoxyzimtsäurenitrils ausgehend von 4-Trifluormethoxyanilin, daß nacheinander diazotiert, iodiert, anschließend stöchiometrich lithiiert wird. Umsetzung des empfindlichen Aryllithiumreagenzes mit Dimethylformamid liefert 4-Trifluormethoxybenzaldehyd, der mit 1-Cyanomethylphosphonsäurediethylester das gewünschte 4-Trifluormethoxyzimtsäurenitril liefert.

Das in EP 174 769 beschriebene Verfahren weist ähnliche Nachteile wie das in EP 0 472 392 beschriebene Verfahren auf. Die große Anzahl von Stufen und die damit einhergehende niedrige Gesamtausbeute sowie die Probleme mit der Verwendung stöchiometrischer Mengen Metallorganika sowie toxischer Cyanomethylphosphonsäureester machen das Verfahren für eine technische Umsetzung nicht geeignet.

Somit bestand ein großer Bedarf 4-Fluoralkoxyzimtsäurenitrile in einfacher und technisch umsetzbarer Weise zugänglich zu machen.

Diese Aufgabe wird gelöst durch ein Verfahren zur Herstellung von 4-Fluoralkoxyzimtsäurenitrilen der Formel (I) 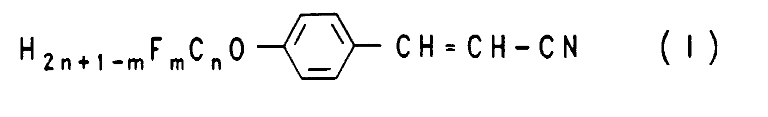 worin n = 1 bis 8 und m = 1 bis 17 sind, wobei m ≤ 2n + 1 ist, dadurch gekennzeichnet, daß man 4-Fluorbenzaldehyd in Gegenwart einer Base und gegebenenfalls eines Lösungsmittels mit einem Fluoralkanol der Formel (II),

H₂ₙ₊₁₋ₘFₘCₙO-H (II)

worin m und n die oben angegebene Bedeutung besitzen, umsetzt und den erhaltenen 4-Fluoralkoxybenzaldehyd mit Cyanessigsäure oder einem Cyanessigsäurealkylester in Gegenwart einer Base und gegebenenfalls eines Lösungsmittels umsetzt.

Von großer Bedeutung ist das Verfahren für die Herstellung von Verbindungen der Formel (I), worin n = 1 bis 4 und m = 2 bis 9 ist.

Außerdem ist die Herstellung von 4-(2,2,3,3-Tetrafluorpropoxy)zimtsäurenitril, 4-Trifluormethoxyzimtsäurenitril, 4-Octafluorpentoxyzimtsäurenitril oder 4-Dodecafluorheptoxyzimtsäurenitril von besonderem Interesse.

Sehr gut geeignet ist das Verfahren auch zur Herstellung teilfluorierter 4-Fluoralkoxyzimtsäurenitrile der Formel (I), worin m < 2n + 1 ist.

Das erfindungsgemäße Verfahren wird wie folgt durchgeführt:
4-Fluorbenzaldehyd wird zusammen mit dem Fluoralkanol in Gegenwart von 0,5 bis 3, insbesondere 0.6 bis 1.25 Äquivalenten Base bei Temperaturen zwischen 10°C und 180°C, insbesondere 60 bis 155°C zum 4-Fluoralkoxybenzaldehyd umgesetzt. Als Base eignen sich z.B. Alkalicarbonate, insbesondere Kaliumcarbonat, Natriumcarbonat oder Gemische aus Kaliumcarbonat und Natriumcarbonat. Die Umsetzung kann sowohl in Gegenwart von dipolar aprotischen Lösungsmitteln wie N,N-Dimethylacetamid, Sulfolan und N,N-Dimethylformamid als auch lösungsmittelfrei durchgeführt werden. Im letzteren Falle wird vorteilhaft in überschüssigem Fluoralkanol gearbeitet, das letztlich als Lösungsmittel fungiert. Die Einsatzmenge an Fluoralkanol ist abhängig von der eingesetzten Menge an Base so zu wählen, daß das Reaktionsgemisch rührbar bleibt und beträgt bezogen auf eingesetzten 4-Fluorbenzaldehyd vorteilhaft 0,8 bis 1,5 Äquivalente.

Das erfindungsgemäße Verfahren kann sowohl mit Teilumsatz als auch bevorzugt mit weitgehend vollständigem Umsatz bezogen auf jeweils eine der beiden Ausgangsmaterialien (4-Fluorbenzaldehyd bzw. Fluoralkanol) bevorzugt 4-Fluorbenzaldehyd gefahren werden. Bei Teilumsatz werden die wertvollen Einsatzkomponenten destillativ zurückgewonnen. Bei vollständigem Umsatz wird ebenfalls das im Überschuß eingesetzte Edukt destillativ zurückgewonnen.

Für die Aufarbeitung bieten sich zwei Wege an:
1) Das Reaktionsgemisch wird durch Filtration von den vorhandenen Salzen (überschüßige Base und gebildetes Basenfluorid) abgetrennt. Anschließend wird das Produkt aus dem Filtrat destilliert.
2) Alternativ dazu ist es möglich durch Zusatz von Wasser das gebildete Basenfluorid und im Überschuß vorhandenes Salz aufzulösen, die gebildeten Phasen zu trennen und das in der organischen Phase befindliche Produkt entweder direkt in die Folgeumsetzung einzubringen oder im Bedarfsfall destillativ zu reinigen. In der wäßrigen Phase befindliches Produkt kann mit Lösungsmitteln wie Toluol, Chlorbenzol, Dichlorbenzol, Methylenchlorid oder tert.Butylmethylether extrahiert werden.

Die Ausbeuten an 4-Fluoralkoxybenzaldehyd nach vorstehendem Verfahren betragen 60 % bis 95 %.

Der erhaltene 4-Fluoralkoxybenzaldehyd wird erfindungsgemäß mit 0,3 bis 4 Äquivalenten, vorzugsweise mit 0,6 bis 2,0 Äquivalenten, bevorzugt mit 0,8 bis 1,2 Äquivalenten Cyanessigsäure oder Cyanessigsäurealkylestern bei Temperaturen von 50 bis 250°C, vorzugsweise 80 bis 180°C in Gegenwart einer Base oder eines basischen Katalysators zum 4-Fluoralkoxyzimtsäurenitril umgesetzt.

Als Cyanessigsäurealkylester haben sich Cyanessigsäuremethylester, Cyanessigsäureethylester und Cyanessigsäurepropylester als günstig erwiesen. Als Base oder basischer Katalysator können aromatische und aliphatische Amine, Alkali- und Erdalkalicarbonate oder basische Oxide und Hydroxide wie NaOH, KOH oder Al₂O₃ eingesetzt werden.

Als Base und gegebenenfalls auch als Lösungsmittel haben sich in vielen Fällen Amine wie Pyridin, Piperidin, Morpholin, Tri-butylamin, Triethylamin, Tripropylamin, Benzylamin, Anilin, Dialkylanilin bewährt.

Die erfindungsgemäße Umsetzung mit Cyanessigsäurederivaten kann in Abwesenheit von sungsmitteln durchgeführt werden. Es kann jedoch vorteilhaft sein, in Anwesenheit von Lösungsmitteln zu arbeiten. Als Lösungsmittel können beispielsweise Aromaten wie Benzol, Toluol, Xylole, dipolar aprotische Lösungsmittel wie Dimethylformamid, Dimethylacetamid, Dimethylsulfoxid, Sulfolan, Alkohole wie Ethanol, Propanol, Butanol, Glyme oder Ether wie Diglyme dienen. Die Menge an Lösungsmittel beträgt, bezogen auf den eingesetzten 4-Fluoralkoxybenzaldehyd 5 bis 90 Gew.-%.

Die Umsetzung kann kontinuierlich oder diskontinuierlich durchgeführt werden. Unter Umständen ist es von Vorteil, Cyanessigsäure oder Cyanessigsäurealkylester während der Reaktion zuzudosieren oder nachzudosieren. Nach beendeter Reaktion wird das Reaktionsgemisch abgekühlt und das Produkt isoliert.

Die Aufreinigung des Rohproduktes kann durch Destillation oder Umkristallisation erfolgen.

Die entstehenden Produkte liegen in der Regel als Gemisch von cis und trans Isomeren bezüglich der Doppelbindung vor. Es ist jedoch möglich, unter besonderen Bedingungen das Isomerenverhältnis zu verändern, so gelingt es beispielsweise durch längeres Erhitzen oder durch Kristallisation bevorzugt eines der Isomere zu erzeugen.

Die Base oder der Basenkatalysator kann prinzipiell homogen als auch heterogen eingesetzt werden.

Unter Umständen ist es vorteilhaft die Reaktion in einem Mehrphasensystem durchzuführen.

Das neue Zwei-Stufen-Verfahren ist dem in EP 0 472 392 und EP 174 769 geschildertem Verfahren deutlich überlegen, da das Produkt in nur zwei Reaktionsschritten verglichen mit sieben Stufen bzw. 5 Stufen erhalten wird. Die Gesamtausbeute an 4-Fluoralkoxyzimtsäurenitril beträgt nach dem erfindungsgemäßen Verfahren ca. 80 % und konnte somit gegenüber EP 0 472 392 deutlich verbessert werden. Dadurch ist das erfindungsgemäße Verfahren dem bisher bekannten alten Verfahren ökonomisch und ökologisch deutlich überlegen.

Die nachstehenden Beispiele dienen zur Erläuterung des erfindungsgemäßen Verfahrens.

### Beispiel 1

103,7 g Kaliumcarbonat, 198,0 g 2,2,3,3-Tetrafluorpropanol und 124 g 4-Fluorbenzaldehyd werden 20 h auf 140°C erhitzt. Anfangs liegt die Siedetemperatur des Reaktionsgemisches bei 125°C. Mit fortschreitender Reaktionszeit steigt die Innentemperatur auf 140°C an. Danach wird auf 100°C gekühlt und überschüssiges 2,2,3,3-Tetrafluorpropanol im Vakuum abdestilliert. Nach Abkühlen auf Raumtemperatur wird das Reaktionsgemisch mit 375 g Wasser versetzt und phasengetrennt. Man erhält 283,8 g einer geblichen, wäßrigen Suspension mit 17 % Wasseranteil und 80 % Tetrafluorpropoxybenzaldehyd. Der Reingehalt des Tetrafluorpropoxybenzaldehyds beträgt 98 %. Die Feuchtware wird direkt weiterverarbeitet. Ausbeute: 227 g 4-(2,2,3,3)-Tetrafluorpropoxybenzaldehyd 98 %ig = 222,5 g 100 %ig = 94 %

### Beispiel 2

165,8 g Kaliumcarbonat, 198,0 g 2,2,3,3-Tetrafluorpropanol und 124 g 4-Fluorbenzaldehyd werden 20 h auf 140°C erhitzt. Anfangs liegt die Siedetemperatur des Reaktionsgemisches bei 125°C. Mit fortschreitender Reaktionszeit steigt die Innentemperatur auf 145°C an.

Bei 45 mm Hg, 130°C Sumpftemperatur und 55°C Kopftemperatur werden anschließend 61 g Ausgangsmaterial abdestilliert. Nach Abkühlen auf Raumtemperatur werden 600 ml Wasser zugesetzt. Nach Phasentrennung erhält man 256,1 g Produkt mit einem Gehalt an 4-(2,2,3,3)-Tetrafluorpropoxybenzaldehyd
(Reingehalt: 97,5 %ig) von 82,5 % (Wassergehalt 14,4 %).
Ausbeute: 89,5 %

### Beispiel 3

500 ml Dimethylacetamid, 304 g Kaliumcarbonat, 248 g 4-Fluorbenzaldehyd und 290 g 2,2,3,3-Tetrafluorpropanol werden 22,5 h auf 140°C erhitzt. Das Reaktionsgemisch wird anschließend auf 20°C abgekühlt und filtriert. Das Salzgemisch wird mit 300 ml Dimethylacetamid gewaschen. Nach Destillation des Filtrates im Vakuum bei 3 mbar, 135°C Sumpf- und 109°C bis 110°C Kopftemperatur erhält man 387,95 g 4-(2,2,3,3-Tetrafluorpropoxy)benzaldehyd mit einem Reingehalt (GC) von 98 % bis 99 %.
Ausbeute: 80,5 %

### Beispiel 4

138,2 g Kaliumcarbonat, 21,2 g Natriumcarbonat, 198 g Tetrafluorpropanol und 124 g 4-Fluorbenzaldehyd werden bei Raumtemperatur unter Argon-Schutzgas vorgelegt und 20 Stunden auf 140°C erhitzt. Anfangs liegt die Siedetemperatur der Reaktionsmischung bei 125°C. Mit fortschreitender Reaktionszeit steigt die Innentemperatur auf 140°C an. Nach 20 Stunden wird auf 25°C abgekühlt und filtriert. Man erhält 229,6 g Filtrat mit einem Gehalt an 4-(2,2,3,3-Tetrafluorpropoxy)benzaldehyd von 72 %. Der Filterkuchen wird 3 mal mit je 100 ml Toluol gewaschen. Filtrat und Waschtoluol werden getrennt gesammelt. Nach Abdestillieren von 63,9 g 2,2,3,3-Tetrafluorpropanol aus dem Filtrat wird der Destillationsrückstand mit dem Waschtoluol vereinigt. Nach Abdestillieren des Toluols bei Normaldruck wird der Rückstand im Vakuum bei 3 mbar und bis zu 135°C Sumpf- und 109°C bis 110°C Kopftemperatur fraktioniert. Man erhält als Hauptlauf 188,7 g 4-(2,2,3,3-Tetrafluorpropoxy)benzaldehyd mit einem Reingehalt (GC) von 98 % bis 99 %.
Ausbeute: 79,9 %

### Beispiel 5

82,9 g Kaliumcarbonat, 63,6 g Natriumcarbonat, 198 g Tetrafluorpropanol und 124 g 4-Fluorbenzaldehyd werden bei Raumtemperatur unter Argon-Schutzgas vorgelegt und 20 Stunden auf 120°C erhitzt. Nach 20 Stunden wird auf 25°C abgekühlt. Anschließend werden bei 20 bis 30 mbar und bis zu 115°C Sumpftemperatur 99,3 g Leichtsieder abdestilliert (ca. 16 Gew.-% 4-Fluorbenzaldehyd, 75,6 % 2,2,3,3-Tetrafluorpropanol). Zum Kolbenrückstand werden 50 ml Toluol zugesetzt und filtriert. Das Salz wird mit 50 ml Toluol gewaschen. Das Waschtoluol wird mit dem Filtrat vereinigt. Nach Abdestillieren des Toluols bei Normaldruck wird der Rückstand im Vakuum bei 3 mbar und bis zu 135°C Sumpf- und 109°C bis 110°C Kopftemperatur fraktioniert. Man erhält 15 g 4-Fluorbenzaldehyd und als Hauptlauf 185,4 g 4-(2,2,3,3-Tetrafluorpropoxy)benzaldehyd mit einem Reingehalt (GC) von 98 % bis 99 %.
Ausbeute: 88,7 %

### Beispiel 6

82,9 g Kaliumcarbonat, 63,6 g Natriumcarbonat, 198 g Tetrafluorpropanol, 124 g 4-Fluorbenzaldehyd und 500 ml N-Methylpyrolidon (NMP) werden bei Raumtemperatur unter Argon-Schutzgas vorgelegt (leicht exotherm) und 20 Stunden auf 120°C erhitzt. Nach 20 Stunden wird auf 25°C abgekühlt und vom Salz abfiltriert. Das Salz wird mit wenig NMP gewaschen. Durch fraktionierte Destillation können aus der NMP-Lösung, die ca. 25 % Gew.-% Produkt enthält 187,5 g 4-(2,2,3,3-Tetrafluorpropoxy)-benzaldehyd mit einem Reingehalt (GC) von 98 % isoliert werden.
Ausbeute: 79,4 %

### Beispiel 7

In einem 250 ml 2-Halskolben werden 36,3 g Kaliumcarbonat, 41,3 g Octafluorpentanol und 33 g 4-Fluorbenzaldehyd bei Raumtemperatur vorgelegt. Anschließend wird auf 140°C erhitzt und 29 Stunden bei 140°C gerührt. Nach Abkühlen auf Raumtemperatur wird abfiltriert. Der Filterrückstand wird 2 mal mit 100 ml Toluol gewaschen. Das Filtrat und Waschtoluol werden vereinigt und fraktioniert.
Man erhält 53 g Octafluorpentoxybenzaldehyd mit einem Reingehalt von 99,5 %.
Ausbeute: 88 %

### Beispiel 8

100,0 g 4-(2,2,3,3-Tetrafluorpropoxy)benzaldehyd und 36,6 g Cyanessigsäure werden in einer Mischung aus 50 ml Pyridin und 50 ml Piperidin 1 Stunde unter Rückfluß erhitzt. Dann gibt man erneut 4,0 g Cyanessigsäure zu und erhitzt weitere 15 Minuten. Nach Abkühlen der Reaktionsmischung wird im Vakuum destilliert. Man erhält 93,03 g des gewünschten Produktes 4-(2,2,3,3-Tetrafluorpropoxyzimt)säurenitril als cis/trans-Gemisch.
Ausbeute: 85 % 4-(2,2,3,3-Tetrafluorpropoxyzimt)säurenitril.

### Beispiel 9

30,0 g 4-(2,2,3,3-Tetrafluorpropoxy)benzaldehyd und 15,3 g Cyanessigsäure werden in 60 ml Piperidin 4 Stunden bei 110°C Ölbadtemperatur erhitzt. Nach Abkühlen der Reaktionsmischung wird im Vakuum destilliert. Man erhält 24,1 g des gewünschten Produktes 4-(2,2,3,3-Tetrafluorpropoxyzimt)säurenitril als cis/trans-Gemisch.
Ausbeute: 74 % 4-(2,2,3,3-Tetrafluorpropoxyzimt)säurenitril.

### Beispiel 10

50,0 g 4-(2,2,3,3-Tetrafluorpropoxy)benzaldehyd, 18,0 g Cyanessigsäure werden in einer Mischung aus 60 ml Pyridin und 5 ml Piperidin 24 Stunden unter Rückfluß erhitzt. Nach Abkühlen der Reaktionsmischung wird im Vakuum destilliert. Man erhält 33,93 g des gewünschten Produktes 4-(2,2,3,3-Tetrafluorpropoxyzimt)säurenitril.
Ausbeute: 62 % 4-(2,2,3,3-Tetrafluorpropoxyzimt)säurenitril.

### Beispiel 11

500,0 g 4-(2,2,3,3-Tetrafluorpropoxy)benzaldehyd, 183 g Cyanessigsäure werden in einer Mischung aus 250 ml Pyridin und 250 ml Piperidin 1 Stunde bei 110°C erhitzt. Dann gibt man erneut 36,6 g Cyanessigsäure zu und erhitzt weitere 30 Minuten. Nach Abkühlen der Reaktionsmischung wird im Vakuum destilliert. Man erhält 433,1 g des gewünschten Produktes 4-(2,2,3,3-Tetrafluorpropoxy)zimtsäurenitril als cis/trans-Gemisch.
Ausbeute: 78 % 4-(2,2,3,3-Tetrafluorpropoxyzimt)säurenitril.

### Beispiel 12

30 g 4-(2,2,3,3-Tetrafluorpropoxy)benzaldehyd, 11 g Cyanessigsäure werden in einer Mischung aus 50 ml Toluol, 10 ml Pyridin und 10 ml Piperidin 30 Minuten bei 110°C erhitzt. Anschließend gibt man erneut 2,2 g Cyanessigsäure zu und erhitzt weitere 30 Minuten. Nach Abkühlen auf Raumtemperatur wird das Rohprodukt aus Ethylacetat umkristallisiert. Man erhält 33 g 4-(2,2,3,3-Tetrafluorpropoxy)zimtsäurenitril (90 %ig)
Ausbeute: 90 % 4-(2,2,3,3-Tetrafluorpropoxy)zimtsäurenitril

## Patentansprüche

1. Verfahren zur Herstellung von Fluoralkoxy-zimtsäurenitrilen der Formel (I) 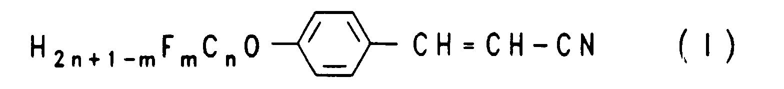 worin n = 1 bis 8 und m = 1 bis 17 sind, wobei m ≤ 2n + 1 ist, dadurch gekennzeichnet, daß man 4-Fluorbenzaldehyd in Gegenwart einer Base und gegebenenfalls eines Lösungsmittels mit einem Fluoralkanol der Formel (II),
H₂ₙ₊₁₋ₘFₘCₙO-H (II)
worin m und n die oben angegebene Bedeutung besitzen, umsetzt und den erhaltenen 4-Fluoralkoxybenzaldehyd mit Cyanessigsäure oder einem Cyanessigsäurealkylester in Gegenwart einer Base und gegebenenfalls eines Lösungsmittels umsetzt.

2. Verfahren nach Anspruch 1, dadurch gekennzeichnet, daß n = 1 bis 4 und m = 2 bis 9 ist.

3. Verfahren nach Anspruch 1, dadurch gekennzeichnet, daß 4-(2,2,3,3-Tetrafluorpropoxy)zimsäurenitril, 4-Trifluormethoxyzimtsäurenitril, 4-Octafluorpentoxyzimtsäurenitril oder 4-Dodecafluorheptoxyzimtsäurenitril hergestellt wird.

4. Verfahren nach Anspruch 1, dadurch gekennzeichnet, daß m < 2n + 1 ist.

5. Verfahren nach mindestens einem der Ansprüche 1 bis 4, dadurch gekennzeichnet, daß die Umsetzung des Fluoralkanols der Formel (II) mit 4-Fluorbenzaldehyd in Gegenwart von 0,5 bis 3,0 Äquivalenten Base bei Temperaturen von 10°C bis 180°C durchgeführt wird.

6. Verfahren nach mindestens einem der Ansprüche 1 bis 5, dadurch gekennzeichnet, daß die Umsetzung des Fluoralkanols der Formel (II) mit 4-Fluorbenzaldehyd in Gegenwart von 0,6 bis 1,25 Äquivalenten Base durchgeführt wird.

7. Verfahren nach mindestens einem der Ansprüche 1 bis 6, dadurch gekennzeichnet, daß die Umsetzung des Fluoralkanols der Formel (II) mit 4-Fluorbenzaldehyd bei Temperaturen von 60 bis 155°C durchgeführt wird.

8. Verfahren nach mindestens einem der Ansprüche 1 bis 7, dadurch gekennzeichnet, daß für die Umsetzung des Fluoralkanols mit 4-Fluorbenzaldehyd als Base ein Alkalicarbonat eingesetzt wird.

9. Verfahren nach mindestens einem der Ansprüche 1 bis 8, dadurch gekennzeichnet, daß für die Umsetzung des Fluoralkanols mit 4-Fluorbenzaldehyd als Base Kaliumcarbonat, Natriumcarbonat oder ein Gemisch aus Natriumcarbonat und Kaliumcarbonat eingesetzt wird.

10. Verfahren nach mindestens einem der Ansprüche 1 bis 9, dadurch gekennzeichnet, daß die Umsetzung des 4-Fluorbenzaldehyds mit Fluoralkanol in Gegenwart eines dipolar aprotischen Lösungsmittels, durchgeführt wird.

11. Verfahren nach mindestens einem der Ansprüche 1 bis 10, dadurch gekennzeichnet, daß die Umsetzung des 4-Fluorbenzaldehyds mit Fluoralkanol in Gegenwart von N,N-Dimethylacetamid, Sulfolan oder N,N-Dimethylformamid als dipolar aprotischem Lösungsmittel durchgeführt wird.

12. Verfahren nach mindestens einem der Ansprüche 1 bis 11, dadurch gekennzeichnet, daß die Umsetzung des 4-Fluorbenzaldehyds mit dem Fluoralkanol in überschüßigem Fluoralkanol als Lösungsmittel durchgeführt wird.

13. Verfahren nach mindestens einem der Ansprüche 1 bis 12, dadurch gekennzeichnet, daß der 4-Fluoralkoxybenzaldehyd mit 0,3 bis 4, Äquivalenten Cyanessigsäure oder Cyanessigsäurealkylester bei Temperaturen von 50°C bis 250°C umgesetzt wird.

14. Verfahren nach mindestens einem der Ansprüche 1 bis 13, dadurch gekennzeichnet, daß der 4-Fluoralkoxybenzaldehyd mit 0,6 bis 2,0 Äquivalenten Cyanessigsäure oder Cyanessigsäurealkylester umgesetzt wird.

15. Verfahren nach mindestens einem der Ansprüche 1 bis 14, dadurch gekennzeichnet, daß der 4-Fluoralkoxybenzaldehyd mit 0,8 bis 1,2 Äquivalenten Cyanessigsäure oder Cyanessigsäurealkylester umgesetzt wird.

16. Verfahren nach mindestens einem der Ansprüche 1 bis 15, dadurch gekennzeichnet, daß der 4-Fluoralkoxybenzaldehyd mit Cyanessigsäure oder Cyanessigsäurealkylester bei Temperaturen von 80°C bis 180°C umgesetzt wird.

17. Verfahren nach mindestens einem der Ansprüche 1 bis 16, dadurch gekennzeichnet, daß die Umsetzung des 4-Fluoralkoxybenzaldehyds mit Cyanessigsäure oder Cyanessigsäurealkylester in Gegenwart eines aromatischen, dipolar aprotischen oder protischen Lösungsmittels durchgeführt wird.

18. Verfahren nach Anspruch 17, dadurch gekennzeichnet, daß als aromatisches Lösungsmittel Benzol, Xylol oder Toluol, als dipolar aprotisches Lösungsmittel Dimethylformamid, Dimethylacetamid, Dimethylsulfoxid oder Sulfolan und als protisches Lösungsmittel Ethanol, Propanol, Butanol oder Glyme verwendet wird.

19. Verfahren nach Anspruch 17 oder 18, dadurch gekennzeichnet, daß 5 bis 90 Gew.-% Lösungsmittel bezogen auf den 4-Fluoralkoxybenzaldehyd eingesetzt werden.

20. Verfahren nach mindestens einem der Ansprüche 1 bis 19, dadurch gekennzeichnet, daß als Base für die Umsetzung des 4-Fluoralkoxybenzaldehyds mit Cyanessigsäure oder Cyanessigsäurealkylester ein Amin, insbesondere Pyridin, Piperidin, Morpholin, Tri-butylamin, Triethylamin, Tripropylamin, Benzylamin, Anilin oder Dialkylanilin oder ein Gemisch davon, eingesetzt wird.

21. Verfahren nach mindestens einem der Ansprüche 1 bis 20, dadurch gekennzeichnet, daß als Cyanessigsäurealkylester Cyanessigsäuremethylester, Cyanessigsäureethylester oder Cyanessigsäurepropylester eingesetzt wird.

## Claims

1. A process for the preparation of fluoroalkoxycinnamonitriles of the formula (I) 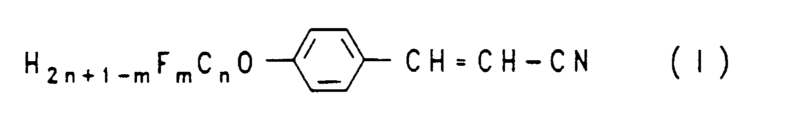 in which n is 1 to 8 and m is 1 to 17, where m ≤ 2n+1, which comprises reacting 4-fluorobenzaldehyde with a fluoroalkanol of the formula (II)
H₂ₙ₊₁₋ₘFₘCₙO-H (II)
in which m and n are as defined above, in the presence of a base and, if appropriate, a solvent, and reacting the resulting 4-fluoroalkoxybenzaldehyde with cyanoacetic acid or an alkyl cyanoacetate in the presence of a base and, if appropriate, a solvent.

2. The process as claimed in claim 1, wherein n is 1 to 4 and m is 2 to 9.

3. The process as claimed in claim 1, wherein 4-(2,2,3,3-tetrafluoropropoxy)cinnamonitrile, 4-trifluoromethoxycinnamonitrile, 4-octafluoropentoxycinnamonitrile or 4-dodecafluoroheptoxycinnamonitrile is prepared.

4. The process as claimed in claim 1, wherein m < 2n+1.

5. The process as claimed in at least one of claims 1 to 4, wherein the reaction of the fluoroalkanol of the formula (II) with 4-fluorobenzaldehyde is carried out in the presence of 0.5 to 3.0 equivalents of base at temperatures from 10°C to 180°C.

6. The process as claimed in at least one of claims 1 to 5, wherein the reaction of the fluoroalkanol of the formula (II) with 4-fluorobenzaldehyde is carried out in the presence of 0.6 to 1.25 equivalents of base.

7. The process as claimed in at least one of claims 1 to 6, wherein the reaction of the fluoroalkanol of the formula (II) with 4-fluorobenzaldehyde is carried out at temperatures from 60°C to 155°C.

8. The process as claimed in at least one of claims 1 to 7, wherein an alkali metal carbonate is employed as the base for the reaction of the fluoroalkanol with 4-fluorobenzaldehyde.

9. The process as claimed in at least one of claims 1 to 8, wherein potassium carbonate, sodium carbonate or a mixture of sodium carbonate and potassium carbonate is employed as the base for the reaction of the fluoroalkanol with 4-fluorobenzaldehyde.

10. The process as claimed in at least one of claims 1 to 9, wherein the reaction of the 4-fluorobenzaldehyde with the fluoroalkanol is carried out in the presence of a dipolar aprotic solvent.

11. The process as claimed in at least one of claims 1 to 10, wherein the reaction of the 4-fluorobenzaldehyde with the fluoroalkanol is carried out in the presence of N,N-dimethylacetamide, sulfolane or N,N-dimethylformamide as the dipolar aprotic solvent.

12. The process as claimed in at least one of claims 1 to 11, wherein the reaction of the 4-fluorobenzaldehyde with the fluoroalkanol is carried out in an excess of fluoroalkanol as the solvent.

13. The process as claimed in at least one of claims 1 to 12, wherein the 4-fluoroalkoxybenzaldehyde is reacted with 0.3 to 4 equivalents of cyanoacetic acid or alkyl cyanoacetate at temperatures from 50°C to 250°C.

14. The process as claimed in at least one of claims 1 to 13, wherein the 4-fluoroalkoxybenzaldehyde is reacted with 0.6 to 2.0 equivalents of cyanoacetic acid or alkyl cyanoacetate.

15. The process as claimed in at least one of claims 1 to 14, wherein the 4-fluoroalkoxybenzaldehyde is reacted with 0.8 to 1.2 equivalents of cyanoacetic acid or alkyl cyanoacetate.

16. The process as claimed in at least one of claims 1 to 15, wherein the 4-fluoroalkoxybenzaldehyde is reacted with cyanoacetic acid or alkyl cyanoacetate at temperatures from 80°C to 180°C.

17. The process as claimed in at least one of claims 1 to 16, wherein the reaction of the 4-fluoroalkoxybenzaldehyde with cyanoacetic acid or alkyl cyanoacetate is carried out in the presence of an aromatic, dipolar aprotic or protic solvent.

18. The process as claimed in claim 17, wherein the aromatic solvent used is benzene, xylene or toluene, the dipolar aprotic solvent used is dimethylformamide, dimethylacetamide, dimethyl sulfoxide or sulfolane, and the protic solvent used is ethanol, propanol, butanol or glyme.

19. The process as claimed in claim 17 or 18, wherein 5 to 90% by weight of solvent, based on the 4-fluoroalkoxybenzaldehyde, are employed.

20. The process as claimed in at least one of claims 1 to 19, wherein the base employed for the reaction of the 4-fluoroalkoxybenzaldehyde with cyanoacetic acid or alkyl cyanoacetate is an amine, in particular pyridine, piperidine, morpholine, tributylamine, triethylamine, tripropylamine, benzylamine, aniline or dialkylaniline, or a mixture of these.

21. The process as claimed in at least one of claims 1 to 20, wherein the alkyl cyanoacetate employed is methyl cyanoacetate, ethyl cyanoacetate or propyl cyanoacetate.

## Revendications

1. Procédé pour la préparation de fluoroalkoxycinnamonitriles de formule (I) 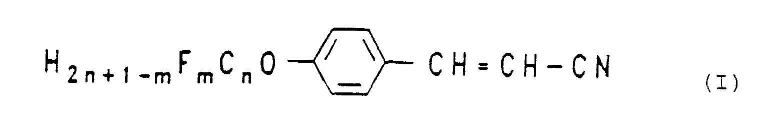 où n = 1 à 8 et m = 1 à 17, m étant ≤2n + 1, caractérisé en ce qu'on fait réagir le 4-fluorobenzaldéhyde en présence d'une base et éventuellement d'un solvant avec un fluoroalcanol de formule (II)
H₂ₙ₊₁₋ₘFₘCₙO-H (II)
où m et n ont les significations ci-dessus, et on fait réagir le 4-fluoroalkoxybenzaldéhyde obtenu avec l'acide cyanoacétique ou un cyanoacétate d'alkyle en présence d'une base et éventuellement d'un solvant.

2. Procédé selon la revendication 1, caractérisé en ce que n = 1 à 4 et m vaut de 2 à 9.

3. Procédé selon la revendication 1, caractérisé en ce qu'on prépare le 4-(2,2,3,3-tétrafluoropropoxy)cinnamonitrile, le 4-trifluorométhoxycinnamonitrile, le 4-octafluoropentoxycinnamonitrile ou le 4-dodécafluoroheptoxycinnamonitrile.

4. Procédé selon la revendication 1, caractérisé en ce que m <2n+1.

5. Procédé selon au moins l'une des revendications 1 à 4, caractérisé en ce que la réaction du fluoroalcanol de formule (II) avec le 4-fluorobenzaldéhyde est réalisé en présence de 0,5 à 3,0 équivalents de base, à une température de 10 °C à 180 °C.

6. Procédé selon au moins l'une des revendications 1 à 5, caractérisé en ce que la réaction du fluoroalcanol de formule (II) avec le 4-fluorobenzaldéhyde est réalisée en présence de 0,6 à 1,25 équivalent de base.

7. Procédé selon au moins l'une des revendications 1 à 6, caractérisé en ce que la réaction du fluoroalcanol de formule (II) avec le 4-fluorobenzaldéhyde est réalisée à une température comprise entre 60 °C et 155 °C.

8. Procédé selon au moins l'une des revendications 1 à 7, caractérisé en ce qu'on utilise un carbonate d'alcalin comme base pour la réaction du fluoroalcanol avec le 4-fluorobenzaldéhyde.

9. Procédé selon au moins l'une des revendications 1 à 8, caractérisé en ce qu'on utilise pour la réaction du fluoroalcanol avec le 4-fluorobenzaldéhyde comme base du carbonate de potassium, du carbonate de sodium ou un mélange de carbonate de potassium et de carbonate de sodium.

10. Procédé selon au moins l'une des revendications 1 à 9, caractérisé en ce que la réaction du fluoroalcanol avec le 4-fluorobenzaldéhyde est réalisé en présence d'un solvant aprotique dipolaire.

11. Procédé selon au moins l'une des revendications 1 à 10, caractérisé en ce que la réaction du 4-fluorobenzaldéhyde avec du fluoroalcanol est réalisée en présence de N,N-diméthylacétamide, de sulfolane ou de N,N-diméthylformamide, en tant que solvants dipolaires aprotiques.

12. Procédé selon au moins l'une des revendications 1 à 11, caractérisé en ce que la réaction du 4-fluorobenzaldéhyde avec le fluoroalcanol ont réalisé en présence d'un excès de fluoroalcanol comme solvant.

13. Procédé selon au moins l'une des revendications 1 à 12, caractérisé en ce qu'on fait réagir le 4-fluoroalkoxybenzaldéhyde avec de 0,3 à 4 équivalents d'acide cyanoacétique ou cyanoacétate d'alkyle à des températures comprises entre 50 et 250 °C.

14. Procédé selon au moins l'une des revendications 1 à 13, caractérisé en ce qu'on fait réagir le 4-fluoroalkoxybenzaldéhyde avec de 0,6 à 2,0 équivalents d'acide cyanoacétique ou cyanoacétate d'alkyle.

15. Procédé selon au moins l'une des revendications 1 à 14, caractérisé en ce qu'on fait réagir le 4-fluoroalkoxybenzaldéhyde avec de 0,8 à 1,2 équivalent d'acide cyanoacétique ou de cyanoacétate d'alkyle.

16. Procédé selon au moins l'une des revendications 1 à 15, caractérisé en ce qu'on fait réagir le 4-fluoroalkoxybenzaldéhyde avec de l'acide cyanoacétique ou de cyanoacétate d'alkyle à des températures comprises entre 80 °C à 180 °C.

17. Procédé selon au moins l'une des revendications 1 à 16, caractérisé en ce que la réaction du le 4-fluoroalkoxybenzaldéhyde avec l'acide cyanoacétique ou le cyanoacétate d'alkyle est réalisée en présence d'un solvant aromatique, dipolaire aprotique ou protique.

18. Procédé selon la revendication 17, caractérisé en ce qu'on utilise comme solvant aromatique le benzène, le xylène ou le toluène, comme solvant dipolaire aprotique le diméthylformamide, le diméthylacétamide, le diméthylsulfoxyde ou le sulfolane, et comme solvant protique l'éthanol, le propanol, le butanol ou le glyme.

19. Procédé selon la revendication 17 ou 18, caractérisé en ce que l'on utilise de 5 à 90 % en poids de solvant par rapport au 4-fluoroalkoxybenzaldéhyde.

20. Procédé selon au moins l'une des revendications 1 à 19, caractérisé en ce qu'on utilise comme base pour la réaction du 4-fluoroalkoxybenzaldéhyde avec l'acide cyanoacétique ou le cyanoacétate d'alkyle une amine, en particulier la pyridine, la pipéridine, la morpholinine, la tributylamine, la triéthylamine, la tripropylamine, la benzylamine, l'aniline ou la dialkylaniline ou leurs mélanges.

21. Procédé selon au moins l'une des revendications 1 à 20, caractérisé en ce qu'on utilise comme cyanoacétate d'alkyle, le cyanoacétate de méthyle, le cyanoacétate d'éthyle ou le cyanoacétate de propyle.
